(19) Europäisches Patentamt
European Patent Office
Office européen des brevets

(11) Veröffentlichungsnummer : **0 363 571 B1**

# (12) EUROPÄISCHE PATENTSCHRIFT

(45) Veröffentlichungstag der Patentschrift :
**17.02.93 Patentblatt 93/07**

(51) Int. Cl.$^5$ : **C07C 311/14,** G03F 7/022

(21) Anmeldenummer : **89111740.0**

(22) Anmeldetag : **28.06.89**

## (54) 1,2-Naphthochinon-2-diazid-sulfonsäureamide und lichtempfindliche Gemische, die diese enthalten.

(30) Priorität : **04.07.88 DE 3822522**

(43) Veröffentlichungstag der Anmeldung :
**18.04.90 Patentblatt 90/16**

(45) Bekanntmachung des Hinweises auf die Patenterteilung :
**17.02.93 Patentblatt 93/07**

(84) Benannte Vertragsstaaten :
**DE FR GB**

(56) Entgegenhaltungen :
**DE-A- 3 325 023**
**DE-A- 3 603 578**
**DE-C- 865 860**
**DE-C- 2 147 947**
**US-A- 3 046 110**
**US-A- 3 687 663**
**US-A- 4 308 368**

(73) Patentinhaber : **HOECHST AKTIENGESELLSCHAFT**
**Postfach 80 03 20**
**W-6230 Frankfurt am Main 80 (DE)**

(72) Erfinder : **Zahn, Wolfgang, Dr. Dipl.-Chem.**
**Goetheweg 3**
**W-6228 Eltville 2 (DE)**
Erfinder : **Buhr, Gerhard, Dr. Dipl.-Chem.**
**Am Erdbeerstein 28**
**W-6240 Königstein (DE)**
Erfinder : **Steppan, Hartmut, Dr. Dipl.-Chem.**
**Panoramastrasse 17**
**W-6200 Wiesbaden (DE)**

**EP 0 363 571 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft neue 1,2-Naphthochinon-2-diazid-sulfonsäureamide sowie lichtempfindliche Gemische und Aufzeichnungsmaterialien, die diese Verbindungen enthalten.

Es ist bereits eine große Anzahl von Derivaten, vor allem aromatischen Estern, von 1,2-Naphthochinon-2-diazid-sulfonsäuren bekannt, die als lichtempfindliche Komponenten in positiv arbeitenden Aufzeichungsmaterialien eingesetzt werden. In dem Buch von Jaromir Kosar: "Light-Sensitive Systems", John Wiley and Sons, New York, 1965, Seiten 343 bis 351, werden eine Reihe von lichtempfindlichen Naphthochinondiaziden beschrieben.

In den üblichen lichtempfindlichen Mischungen werden die Naphthochinondiazide mit polymeren Bindemitteln kombiniert, die phenolische Hydroxylgruppen tragen. Im allgemeinen sind diese Bindemittel Novolake, die den aus den lichtempfindlichen Mischungen hergestellten Schichten mechanische Festigkeit und Resistenz, aber auch eine gewisse Sprödigkeit verleihen.

Um diese Sprödigkeit zu verringern, ist es notwendig, den Anteil an Novolaken in den lichtempfindlichen Gemischen zu reduzieren oder wenn möglich, vollständig auf Novolake als Schichtbestandteile zu verzichten. Dadurch werden auch eventuell auftretende Mischungs- und Löslichkeitsprobleme umgangen.

Monomere Naphthochinondiazide bilden allein keine völlig homogenen Schichten und kommen daher als Beschichtungsmaterial nicht in Betracht. Um auf Bindemittel in diesen lichtempfindlichen Mischungen verzichten zu können, müssen daher polymere Naphthochinondiazid-Derivate eingesetzt werden.

Es ist bereits eine Reihe von polymeren Naphthochinondiaziden bekannt, wobei der Naphthochinondiazidrest über eine Estergruppierung an das polymere Grundgerüst oder an entsprechend funktionalisierte Seitenketten des Polymeren gebunden ist. So werden in der DE-C-865 860 und der EP-B-0 055 814 Ester von Naphthochinondiazid-sulfonsäuren mit Phenolharzen beschrieben. Ähnliche Verbindungen sind in den JP-A-86/267043, 86/266423, 85/138544 und 87/244039 und der US-A-4,308,368 beschrieben. In der JP-A-86/245154 werden Umsetzungsprodukte von Naphthochinondiazidsulfonylchloriden mit Polyurethanen beschrieben.

Die EP-A-0 231 855 beschreibt Bis-naphthochinondiazid-sulfonsäureamide von sekundären Diaminen, die besonders im mittleren UV-Bereich empfindlich sind.

Bei den bisher genannten Beispielen wird, soweit es sich um polymere Verbindungen handelt, der Naphthochinondiazidrest in einer polymeranalogen Reaktion an ein vorgefertigtes Polymergerüst gebunden, wobei alle bekannten Nachteile von polymeranalogen Reaktionen zu beachten sind. Es ist deshalb günstiger, den Einbau der Naphthochinondiazidgruppe mit der Polymerisationsreaktion zu koppeln, d.h. geeignete Naphthochinondiazid-Verbindungen als Monomerbausteine zu nutzen.

Aufgabe der Erfindung war es daher, neue monomere und polymere Verbindungen mit seitenständigen Naphthochinondiazidresten bereitzustellen, die eine genauere Dosierung der im Polymermolekül enthaltenen Naphthochinondiazidreste ermöglichen und die zur Herstellung lichtempfindlicher Gemische ohne Zusatz von Bindemitteln geeignet sind.

Gegenstand der Erfindung sind Verbindungen der allgemeinen Formel I

$$R^2-CH_2-\underset{}{\overset{D}{\overset{|}{N}}}-CH_2-R^1-OH \qquad (I)$$

worin

D einen 1,2-Naphthochinon-2-diazid-4-sulfonyl- oder -5-sulfonylrest,

$R^1$ eine Alkylengruppe mit 1 bis 3 Kohlenstoffatomen,

$R^2$ ein Wasserstoffatom, eine Alkylgruppe mit 1 bis 3 Kohlenstoffatomen oder eine Gruppe $R^1$-OH bedeutet,

oder der allgemeinen Formel II

$$\left[-O-R^1-CH_2-\overset{D}{\overset{|}{N}}-CH_2-R^1-O-\right]_n \left[-CO-X-CO-\right]_{n+m-1} \left[-O-R^3-O-\right]_m \qquad (II)$$

worin

X eine Alkylengruppe mit 2 bis 12 Kohlenstoffatomen, eine Arylengruppe mit 6 bis 10 Kohlenstoffatomen oder eine Gruppe der Formel

NH-Y-NH,

worin

Y eine Alkylengruppe mit 2 bis 12 Kohlenstoffatomen oder eine Arylengruppe mit 6 bis 14 Kohlenstoffatomen ist,

$R^3$ eine Alkylengruppe mit 2 bis 12 Kohlenstoffatomen, die ggf. durch Ethersauerstoffatome unterbrochen ist,

n eine Zahl von 1 bis 40 und

m eine Zahl von 0 bis 50 bedeutet,

wobei das Verhältnis m:(m+n) 0:100 bis 95:100 beträgt und $R^1$ und D die oben angegebene Bedeutung haben.

Erfindungsgemäß wird ferner ein lichtempfindliches Gemisch vorgeschlagen, das als wesentliche Bestandteile ein polymeres, wasserunlösliches, in organischen Lösemitteln und in wäßrig-alkalischen Lösungen lösliches Bindemittel und ein 1,2-Naphthochinon-2-diazidsulfon-säureamid enthält. Das erfindungsgemäße Gemisch ist dadurch gekennzeichnet, daß es 5 bis 100 Gew.-%, bezogen auf seine nichtflüchtigen Bestandteile, an Verbindungen gemäß Anspruch 1 enthält.

Schließlich wird erfindungsgemäß auch ein lichtempfindliches Aufzeichnungsmaterial mit einem Schichtträger und einer lichtempfindlichen Schicht vorgeschlagen.

Das erfindungsgemäße Aufzeichnungsmaterial ist dadurch gekennzeichnet, daß die lichtempfindliche Schicht eine Verbindung der Formeln I und II enthält.

Die erfindungsgemäßen monomeren Verbindungen der Formel I können als solche in lichtempfindlichen Gemischen eingesetzt werden, wobei es zweckmäßig ist, dem Gemisch in üblicher Weise ein alkalilösliches polymeres Bindemittel zuzusetzen. Sie sind insbesondere auch als Zwischenprodukte zur Herstellung der polymeren Verbindungen der Formel II geeignet. Diese Polymeren lassen sich durch gezielten, dosierten Einbau von Diolkomponenten HO-$R^3$-OH in ihren Eigenschaften auf den gewünschten Verwendungszweck abstimmen. Je nach Bedarf kann auf den Einbau von neutralen Diolkomponenten ganz verzichtet werden, oder die polymeren Naphthochinondiazide können mit nicht lichtempfindlichen Bindemitteln kombiniert werden.

Von den Verbindungen der allgemeinen Formel I werden diejenigen mit $R^2$ = Hydroxyalkyl bevorzugt, wenn sie zu Polymeren weiterverarbeitet werden sollen. $R^1$ kann ein geradkettiger oder verzweigter Rest sein und hat bevorzugt 1 oder 2 Kohlenstofftome. Als Alkylgruppen $R^2$ werden Methylgruppen bevorzugt.

In der allgemeinen Formel II ist Y bevorzugt eine Alkylengruppe mit 4 bis 10, insbesondere 6 bis 10 Kohlenstoffatomen; als Arylengruppen werden einkernige Arylengruppen bevorzugt. Beispiele für geeignete Diisocyanate OCN-X-NCO sind Ethylendiisocyanat, Butylendiisocyanat, Hexamethylendiisocyanat, 2,2,4-Trimethylhexamethylendiisocyanat, Cyclohexylendiisocyanat, Isophorondiisocyanat, Tolylendiisocyanat, Diphenylmethandiisocyanat und Naphthylendiisocyanat.

Wenn X eine Alkylengruppe ist, hat diese vorzugsweise 4 bis 10 Kohlenstoffatome, als Arylengruppen werden Phenylgruppen bevorzugt. $R^3$ hat bevorzugt 4 bis 10, insbesondere 4 bis 8 Kohlenstoffatome. Beispiele für geeignete Diole HO-$R^3$-OH sind Ethylenglykol, Propylenglykol, Butandiol-1,4, Hexamethylendiol, Neopentylglykol, 1,1,3-Trimethyl-propandiol-1,3, Diethylenglykol, Triethylenglykol, Pentaethylenglykol und Tripropylenglykol. Das Verhältnis von m:(m+n) liegt vorzugsweise im Bereich von 10 bis 95:100, insbesondere von 25 bis 90:100.

Hergestellt werden die erfindungsgemäßen Verbindungen der Formel I nach bekannten Verfahren durch Umsetzung von Bis-hydroxyalkylaminen mit reaktiven Naphthochinondiazid-sulfonsäurederivaten, etwa dem Säurechlorid.

Als bevorzugte Verfahren kommen die Umsetzungen in inerten Lösemitteln, wie Ketonen oder chlorierten Kohlenwasserstoffen, in Gegenwart von anorganischen oder organischen Basen, wie Natriumcarbonat oder tertiären Aminen wie Triethylamin, in Betracht. Die erfindungsgemäßen Naphthochinondiazid-sulfonsäureamide können jedoch auch unter den Bedingungen der Phasen-Transfer-Katalyse, z.B. im Gemisch von Methylenchlorid mit einer wäßrigen Lösung von Natriumcarboant oder Tetraalkylammoniumhydroxid, mit einem geeigneten Katalysator, wie Tetrabutylammoniumbromid, hergestellt werden.

Im folgenden wird eine allgemeine Vorschrift zur Herstellung der monomeren erfindungsgemäßen Naphthochinondiazidsulfonsäureamide gegeben:

0,2 mol 1,2-Naphthochinon-2-diazid-sulfonsäurechlorid und 0,22 mol Natriumcarbonat oder Triethylamin in 400 ml Aceton werden unter Rühren tropfenweise mit 0,2 mol Dialkanolamin versetzt, wobei man die Temperatur durch Kühlen unter 25° C hält. Nach Beendigung des Zutropfens wird noch 1 Stunde weitergerührt und

a) falls das Naphthochinondiazid-sulfonsäureamid bereits ausgefallen ist, dieses abgesaugt, mit kaltem

Aceton und anschließend mit Wasser gewaschen und der Filterrückstand bei vermindertem Druck oder im Umluft-Trockenschrank bei mäßig erhöhter Temperatur getrocknet,
oder
b) bei im Reaktionsgemisch vollständig löslichem Naphthochinondiazid-sulfonsäureamid wird der Ansatz durch Absaugen von anorganischen Bestandteilen befreit. Das Filtrat wird in die zehnfache Menge 4%iger wäßriger Salzsäure eingerührt, das ausgefallene Sulfonsäureamid abfiltriert, mit Wasser säurefrei gewaschen und wie unter a) getrocknet.

Die Umsetzungen der monomeren Naphthochinondiazid-sulfonsäureamide, die zwei Hydroxylgruppen besitzen, mit bifunktionellen Verbindungen wie Dicarbonsäuredichloriden oder Diisocyanaten erfolgt nach bekannten Verfahren.

Die Reaktionen mit Dicarbonsäuredichloriden werden bevorzugt in inerten Lösemitteln, wie Ketonen oder chlorierten Kohlenwasserstoffen, in Gegenwart von anorganischen oder organischen Basen, wie Kaliumcarbonat oder Triethylamin, bei Raumtemperatur oder leicht erhöhter Temperatur durchgeführt. Nach beendeter Reaktion wird die Reaktionslösung i.a. von unlöslichen Bestandteilen getrennt und in einen großen Überschuß an 4%iger wäßriger Salzsäure gegossen. Anschließend saugt man das ausgefallene Produkt ab, wäscht neutral und trocknet den Filterrückstand bei vermindertem Druck oder im Umluft-Trockenschrank bei mäßig erhöhter Temperatur.

Die Umsetzungen der monomeren Naphthochinondiazidsulfonsäure-bis-hydroxyalkyl-amide mit Diisocyanaten erfolgen bevorzugt in inerten Lösemitteln, wie Ethern oder Ketonen, in Gegenwart von geeigneten Katalysatoren, wie Dibutylzinndilaurat oder Diazabicyclooctan, bei Raumtemperatur oder mäßig erhöhter Temperatur. Die Aufarbeitung und Isolierung der Polyurethane geschieht analog zu den o.g. Polyestern.

Bei den genannten Umsetzungen kann das monomere Naphthochinondiazid-diol teilweise durch eine oder mehrere andere Diolkomponenten ersetzt werden, wobei im übrigen die Verfahrensweise die gleiche bleibt.

Die bei vielen Positivmaterialien auf Basis von Naphthochinondiaziden eingesetzten Bindemittel können auch bei den erfindungsgemäßen Gemischen mit den neuen Naphthochinondiaziden zugesetzt werden. Die Art und Menge der Bindemittel kann je nach Anwendungszweck verschieden sein. Bevorzugt sind Bindemittelanteile am Gesamtfeststoff zwischen 0 % und 95 %, besonders bevorzugt 0 - 80 %. Als Bindemittel geeignet sind Novolake und polymere Bindemittel mit seitenständigen phenolischen Hydroxylgruppen, wie die Polymeren der Vinylphenole oder der Ester und Amide von Acrylsäure und Methacrylsäure, z.B. mit Hydrochinon, Brenzkatechin, Resorcin, Pyrogallol oder Aminophenolen. Neben den Homopolymeren können auch Copolymere der genannten Monomeren untereinander oder auch mit anderen polymerisierbaren Monomeren, wie Styrol, Methylmethacrylat, Methylacrylat, Biphenylylmethacrylat oder Biphenylylacrylat als Bindemittel in den erfindungsgemäßen Gemischen verwendet werden. Auch Mischungen der Polymerisate mit Novolaken sind einsetzbar.

Zur Beschichtung eines geeigneten Schichtträgers für die Herstellung des erfindungsgemäßen Aufzeichnungsmaterials werden die Gemische im allgemeinen in einem Lösemittel gelöst. Die Wahl der Lösemittel ist auf das vorgesehene Beschichtungsverfahren, die Schichtdicke und die Trocknungsbedingungen abzustimmen. Geeignete Lösemittel für das erfindungsgemäße Gemisch sind Ketone, wie Butanon oder N-Methyl-pyrrolidon; Alkoholether wie 2-Ethoxy-ethanol oder 1-Ethoxy-propan-2-ol, Alkoholether-acetate wie 2-Ethoxyethylacetat oder 2-Ethoxy-propylacetat; und Ester wie Butylacetat. Es können auch Gemische von Lösemitteln verwendet werden, die u.a. auch Xylol enthalten können. Prinzipiell sind alle Lösemittel verwendbar, die mit den Schichtkomponenten nicht irreversibel reagieren.

In geringen Mengen (bis zu 2 %) kann das lichtempfindliche Gemisch für spezielle Erfordernisse wie Haftung, Glätte der Oberfläche, spezielle Absorptionseigenschaften etc. noch Substanzen wie Netzmittel, Haftvermittler und Farbstoffe enthalten. Weiterhin können dem lichtempfindlichen Gemisch auch nichtpolymere Naphthochinondiazidderivate zugesetzt werden.

Als Träger für das lichtempfindliche Gemisch eignen sich alle üblicherweise in lithographischen Prozessen eingesetzten Materialien. Als Beispiele seien genannt Metalle, wie Aluminium-Träger für Offsetdruckplatten, die eine geeignete Vorbehandlung erfahren haben, oder mit Kupfer kaschierte Isolierstoffplatten für die Leiterplattenfertigung, Kunststoffolien, die auch als intermediärer Schichtträger dienen können, und die bei der Herstelung von mikroelektronischen Bauelementen verwendeten Träger und Oberflächen, z.B. Silicium, das auch oberflächlich oxydiert oder mit geeigneten Dotierungsmaterialien implantiert sein kann, Siliciumnitrid, Polysilicium, Polyimide oder Metalle wie Aluminium.

Die Beschichtung des Trägermaterials erfolgt in bekannter Weise durch Aufschleudern, Sprühen, Walzen, Tauchen, mittels Breitschlitzdüsen, Rakeln oder durch Gießerantrag.

Belichtet wird mit den üblichen Lichtquellen, z.B. Quecksilberdampflampen, die auch mit Metallhalogeniden dotiert sein können. Geeignet sind auch Strahlungsquellen, die energiereichere Strahlung emittieren, z.B. Laser oder Belichtungsgeräte mit Röntgen- oder Elektronenstrahlen.

Die zum Entwickeln verwendeten wäßrig-alkalischen Lösungen entfernen die vom Licht getroffenen Stellen der lichtempfindlichen Schicht und erzeugen so ein positives Abbild der Vorlage. Geeignete Entwickler sind wäßrig-alkalische Lösungen, die metallionenfrei sind, aber auch Metallionen wie Natrium- und/oder Kaliumionen enthalten können. Die Entwicklerlösungen können gepuffert sein, zum Beispiel mit Silikat-, Borat- oder Phosphatlösungen oder geeigneten Mischungen von Salzlösungen, und können auch geringe Mengen von Tensiden und Lösemitteln enthalten.

Anwendung finden die erfindungsgemäßen strahlungsempfindlichen Gemische bei lithographischen Prozessen, z.B. bei der Herstellung von integrierten Schaltungen oder von diskreten elektronischen Bausteinen. Sie dienen dabei als Maskierungsmaterial für verschiedene Prozeßschritte, z.B. beim Ätzen des Schichtträgers, bei der Implantation von Ionen in den Schichtträger oder der Abscheidung von Materialien auf den Schichtträger. Die erfindungsgemäßen strahlungsempfindlichen Gemische sind auch geeignet für die Herstellung von Druckformen und können bei der Fertigung von Leiterplatten eingesetzt werden.

Im folgenden werden Beispiele für erfindungsgemäße 1,2-Naphthochinon-2-diazid-sulfonsäureamide und erfindungsgemäße lichtempfindliche Gemische beschrieben. Dabei werden zunächst in den Tabellen I bis V erfindungsgemäße Verbindungen angegeben, die entsprechend der oben angegebenen allgemeinen Verfahrensweise hergestellt wurden. In den Anwendungsbeispielen 1 bis 13 werden eine Anzahl dieser Verbindungen als lichtempfindliche Substanzen in positiv arbeitenden lichtempfindlichen Aufzeichnungsmaterialien eingesetzt. In den Beispielen sind die Mengen in der Regel in Gewichtsteilen (Gt) angegeben. Mengenverhältnisse und Prozentangaben sind, wenn nichts anderes angegeben ist, in Gewichtseinheiten zu verstehen.

## Tabelle I

Verbindungen der Formel I mit D = 1,2-Naphthochinon-2-diazid-5-sulfonyl

| Verbindung Nr. | $R^1$ | $R^2$ | | Analyse (%) ber. | gef. |
|---|---|---|---|---|---|
| 1 | $CH_2$ | $CH_2OH$ | C | 49,84 | 49,6 |
| | | | H | 4,48 | 4,6 |
| | | | N | 12,46 | 12,3 |
| | | | S | 9,50 | 9,5 |
| 2 | $CH(CH_3)$ | $CH(CH_3)OH$ | C | 52,59 | 52,6 |
| | | | H | 5,24 | 5,4 |
| | | | N | 11,50 | 11,5 |
| | | | S | 8,77 | 8,6 |

## Tabelle II

Verbindungen der Formel II mit D = 1,2-Naphthochinon-2-diazid-5-sulfonyl und m = 0 **n = 1-40**

| Verbindung Nr. | $R^1$ | X |
|---|---|---|
| 3 | $CH_2$ | $(CH_2)_4$ |
| 4 | " | $(CH_2)_7$ |
| 5 | " | $(CH_2)_8$ |
| 6 | " | $(CH_2)_{10}$ |
| 7 | " | 1,4-Phenylen |
| 8 | $CH(CH_3)$ | $(CH_2)_4$ |
| 9 | " | $(CH_2)_7$ |
| 10 | " | $(CH_2)_8$ |
| 11 | " | $(CH_2)_{10}$ |

## Tabelle III

Verbindungen der Formel II mit D = 1,2-Naphthochinon-2-diazid-5-sulfonyl, X = NH-Y-NH und m = 0 **n = 1-40**

| Verbindung Nr. | $R^1$ | Y |
|---|---|---|
| 12 | $CH_2$ | $(CH_2)_6$ |
| 13 | " | $CH_2-C(CH_3)_2-CH_2-CH(CH_3)-(CH_2)_2$ |
| 14 | $CH(CH_3)$ | $(CH_2)_6$ |
| 15 | " | $CH_2-C(CH_3)_2-CH_2-CH(CH_3)-(CH_2)_2$ |

Tabelle IV

Verbindungen der Formel II mit D = 1,2-Naphthochinon-2-diazid-5-sulfonyl, $R^1$ = $CH(CH_3)$ X = NH-Y-NH und Y = $(CH_2)6$

| Verbindung Nr. | $R^3$ | Molverhältnis n/m |
|---|---|---|
| 16 | $(CH_2)_4$ | 3:1 |
| 17 | " | 1:1 |
| 18 | " | 1:4 |
| 19 | $(CH_2)_6$ | 1:4 |
| 20 | $CH_2C(CH_3)_2CH_2$ | 1:1 |
| 21 | " | 1:1,5 |
| 22 | " | 1:2 |
| 23 | " | 1:3 |
| 24 | " | 1:5 |
| 25 | $C(CH_3)_2CH_2CH(CH_3)$ | 1:1 |
| 26 | " | 1:1,5 |
| 27 | " | 1:2 |
| 28 | " | 1:5 |
| 29 | $(CH_2)_2$-O-$(CH_2)_2$-O-$(CH_2)_2$ | 1:1 |

Tabelle V

Verbindungen der Formel II mit D = 1,2-Naphthochinon-2-diazid-5-sulfonyl, $R^1$ = CH($CH_3$) und X = NH-Y-NH
Y = 2,2,4-Trimethyl-hexamethylen

| Verbindung Nr. | $R^3$ | Molverhältnis n/m |
|---|---|---|
| 30 | $(CH_2)_4$ | 3:1 |
| 31 | " | 1:1 |
| 32 | " | 1:4 |
| 33 | " | 1:9 |
| 34 | $(CH_2)_6$ | 1:1 |
| 35 | " | 1:4 |
| 36 | $CH_2C(CH_3)_2CH_2$ | 1:1 |
| 37 | " | 1:1,5 |
| 38 | " | 1:2 |
| 39 | " | 1:5 |
| 40 | $C(CH_3)_2CH_2CH(CH_3)$ | 1:1 |
| 41 | " | 1:1,5 |
| 42 | " | 1:2 |
| 43 | " | 1:5 |
| 44 x) | $(CH_2)_6$ | 1:1 |

x) D = 1,2-Naphthochinon-2-diazid-4-sulfonyl

Beispiel 1

Auf einen elektrolytisch aufgerauhten und anodisierten Aluminium-Schichtträger wird eine Beschichtungslösung aus

4,8 Gt Verbindung 2 und
95,2 Gt Butanon

aufgebracht, so daß sich nach dem Trocknen ein Schichtgewicht von 1,20 g/m² ergibt. Nach dem Trocknen wird die beschichtete Druckplatte unter einer Vorlage, die neben Strich- und Rastermotiven einen Halbtonstufenkeil mit 13 Stufen der optischen Dichte von je 0,15 enthält, mit einer 5 kW-Metallhalogenidlampe im Abstand von 120 cm 90 Sekunden belichtet und mit einem Entwickler aus

3,7 Gt Natriummetasilikat x 9 $H_2O$ und
0,1 Gt Natrium-laurylether-polyglykolsulfat in
96,2 Gt Wasser

8 Sekunden entwickelt, wobei die belichteten Schichtbereiche entfernt werden. Man erhält eine Druckform, die im Halbtonkeil 4 offene Keilstufen aufweist.

Beispiel 2

Auf einen elektrolytisch aufgerauhten und anodisierten Aluminium-Schichtträger wird eine Beschichtungslösung aus

12,3 Gt Verbindung 12 und
6,1 Gt Verbindung 6 in
81,6 Gt Tetrahydrofuran

aufgebracht, so daß sich nach dem Trocknen ein Schichtgewicht von 1,28 g/m$^2$ ergibt. Nach dem Trocknen wird die beschichtete Druckplatte unter der in Beispiel 1 angegebenen Vorlage mit einer 5 kW-Metallhalogenidlampe im Abstand von 120 cm 150 Sekunden belichtet und mit einem Entwickler aus

5,2 Gt Natriummetasilikat x 9 $H_2O$ und
0,1 Gt Netzmittel wie in Beispiel 1 in
94,7 Gt Wasser

1 Minute entwickelt, wobei die belichteten Schichtbereiche entfernt werden. Man erhält eine Druckform, die im Halbtonkeil 3 offene Keilstufen aufweist.

Beispiel 3

Auf einen elektrolytisch aufgerauhten und anodisierten Aluminium-Schichtträger wird eine Beschichtungslösung aus

2,2 Gt Verbindung 3 und
1,1 Gt Verbindung 12 in
96,6 Gt Tetrahydrofuran

aufgebracht, so daß sich nach dem Trocknen ein Schichtgewicht von 0,97 g/m$^2$ ergibt. Die beschichtete Druckplatte wird wie in Beispiel 2 belichtet und mit einem Entwickler aus

2,1 Gt Natriumhydroxid und
1,5 Gt Borsäure in
96,4 Gt Wasser

1 Minute entwickelt, wobei die belichteten Schichtbereiche entfernt werden. Man erhält eine Druckform, die im Halbtonkeil 4 offene Keilstufen aufweist.

Beispiel 4

Auf einen elektrolytisch aufgerauhten und anodisierten Aluminium-Schichtträger wird eine Beschichtungslösung aus

3,85 Gt Verbindung 13 in
96,15 Gt 2-Methoxy-ethanol

aufgebracht, so daß sich nach dem Trocknen ein Schichtgewicht von 0,63 g/m$^2$ ergibt. Die beschichtete Druckplatte wird wie in Beispiel 2 belichtet und mit einem Entwickler aus

6,5 Gt Natriummetasilikat x 9 $H_2O$ und
0,1 Gt Netzmittel wie in Beispiel 1 in
93,4 Gt Wasser

1 Minute entwickelt, wobei die belichteten Schichtbereiche entfernt werden. Man erhält eine Druckform, die im Halbtonkeil 4 offene Keilstufen aufweist.

Beispiel 5

Auf einen elektrolytisch aufgerauhten und anodisierten Aluminium-Schichtträger wird eine Beschichtungslösung aus

5,7 Gt Verbindung 8 in
96,3 Gt Butanon

aufgebracht, so daß sich nach dem Trocknen ein Schichtgewicht von 1,15 g/m$^2$ ergibt. Die beschichtete Druckplatte wird wie in Beispiel 1, aber 75 Sekunden belichtet und mit einem Entwickler aus 0,43 Gt Natriummetasilikat x 9 $H_2O$ und

0,1 Gt Netzmittel wie in Beispiel 1 in

99,47 Gt Wasser
15 Sekunden entwickelt, wobei die belichteten Schichtbereiche entfernt werden. Man erhält eine Druckform, die im Halbtonkeil 4 offene Keilstufen aufweist.

Beispiel 6

Auf einen elektrolytisch aufgerauhten und anodisierten Aluminium-Schichtträger wird eine Beschichtungslösung aus
3,1 Gt Verbindung 14 in
96,9 Gt Butanon
aufgebracht, so daß sich nach dem Trocknen ein Schichtgewicht von 0,66 g/m$^2$ ergibt. Die beschichtete Druckplatte wird wie in Beispiel 1 belichtet und mit einem Entwickler aus
7,2 Gt Natriummetasilikat x 9 $H_2O$ und
0,1 Gt Netzmittel wie in Beispiel 1 in
92,7 Gt Wasser
5 Sekunden entwickelt, wobei die belichteten Schichtbereiche entfernt werden. Man erhält eine Druckform, die im Halbtonkeil 4 offene Keilstufen aufweist.

Beispiel 7

Auf einen elektrolytisch aufgerauhten und anodisierten Aluminium-Schichtträger wird eine Beschichtungslösung aus
3,85 Gt Verbindung 15 in
96,15 Gt Butanon
aufgebracht, so daß sich nach dem Trocknen ein Schichtgewicht von 0,91 g/m$^2$ ergibt. Die beschichtete Druckplatte wird wie in Beispiel 1, aber 100 Sekunden belichtet und mit einem Entwickler aus
6,5 Gt Natriummetasilikat x 9 $H_2O$ und
0,1 Gt Netzmittel wie in Beispiel 1 in
93,4 Gt Wasser
1 Minute entwickelt, wobei die belichteten Schichtbereiche entfernt werden. Man erhält eine Druckform, die im Halbtonkeil 2 offene Keilstufen aufweist.

Beispiel 8

Auf einen elektrolytisch aufgerauhten und anodisierten Aluminium-Schichtträger wird eine Beschichtungslösung aus
3,85 Gt Verbindung 25 in
96,15 Gt Butanon
aufgebracht, so daß sich nach dem Trocknen ein Schichtgewicht von 0,92 g/m$^2$ ergibt. Die beschichtete Druckplatte wird wie in Beispiel 1, aber 85 Sekunden belichtet und mit einem Entwickler aus
5,3 Gt Tetramethylammoniumhydroxid in
94,7 Gt Wasser
1 Minute entwickelt, wobei die belichteten Schichtbereiche entfernt werden. Man erhält eine Druckform, die im Halbtonkeil 3 offene Keilstufen aufweist.

Beispiel 9

Auf einen elektrolytisch aufgerauhten und anodisierten Aluminium-Schichtträger wird eine Beschichtungslösung aus
3,85 Gt Verbindung 36 in
96,15 Gt Butanon
aufgebracht, so daß sich nach dem Trocknen ein Schichtgewicht von 0,87 g/m$^2$ ergibt. Die beschichtete Druckplatte wird wie in Beispiel 8 belichtet und mit dem in Beispiel 8 angegebenen Entwickler 1 Minute entwickelt, wobei die belichteten Schichtbereiche entfernt werden. Man erhält eine Druckform, die im Halbtonkeil 3 offene Keilstufen aufweist.

Beispiel 10

Auf einen elektrolytisch aufgerauhten und anodisierten Aluminium-Schichtträger wird eine Beschichtungslösung aus

3,85 Gt Verbindung 23 in
96,15 Gt Butanon

aufgebracht, so daß sich nach dem Trocknen ein Schichtgewicht von 0,90 g/m² ergibt. Die beschichtete Druckplatte wird wie in Beispiel 1, aber 70 Sekunden belichtet und mit dem in Beispiel 4 angegebenen Entwickler 1 Minute entwickelt, wobei die belichteten Schichtbereiche entfernt werden. Man erhält eine Druckform, die im Halbtonkeil 4 offene Keilstufen aufweist. Ein ähnliches Ergebnis erhält man, wenn man in der genannten Rezeptur die Verbindung 23 durch jeweils die gleiche Menge einer der Verbindungen 28 oder 29 ersetzt.

Beispiel 11

Auf einen elektrolytisch aufgerauhten und anodisierten Aluminium-Schichtträger wird eine Beschichtungslösung aus

1,9 Gt Verbindung 15 und
1,9 Gt Polyvinylphenol (Maruzen Oil) in
38,5 Gt Butanon und
57,7 Gt 2-Methoxy-ethanol

aufgebracht, so daß sich nach dem Trocknen ein Schichtgewicht von 1,00 g/m² ergibt. Die beschichtete Druckplatte wird wie in Beispiel 8 belichtet und mit dem in Beispiel 4 angegebenen Entwickler 1 Minute entwickelt, wobei die belichteten Schichtbereiche entfernt werden. Man erhält eine Druckform, die im Halbtonkeil 4 offene Keilstufen aufweist.

Beispiel 12

Eine Silicium-Scheibe wird mit einer Beschichtungslösung, bestehend aus

4,6 Gt Verbindung 1 und
15,7 Gt Kresol-Formaldehyd-Novolak (Schmelzbereich 122 - 132° C nach DIN 53181) in
30,9 Gt Tetrahydrofuran und
48,8 Gt N-Methylpyrrolidon,

schleuderbeschichtet, so daß sich nach dem Trocknen eine Schichtdicke von 1,8 µm ergibt. Die so erhaltene Schicht besitzt gute Lichtempfindlichkeit. Der geeignete Entwickler (eine 0,14 normale, gepufferte NaOH-Lösung) ist gegenüber herkömmlichen Entwicklern stark verdünnt und ergibt eine geringere Abwasserbelastung.

Ein ähnliches Ergebnis wird erhalten, wenn man in der genannten Rezeptur die Verbindung 1 durch Verbindung 2 ersetzt.

Beispiel 13

Auf einen elektrolytisch aufgerauhten und anodisierten Aluminium-Schichtträger wird eine Beschichtungslösung aus

3,9 Gt Verbindung 44 in
96,1 Gt 2-Methoxy-ethanol

aufgebracht, so daß sich nach dem Trocknen ein Schichtgewicht von 0,72 g/m² ergibt. Die beschichtete Druckplatte wird wie in Beispiel 2 belichtet und mit dem in Beispiel 4 angegebenen Entwickler 1 Minute entwickelt, wobei die belichteten Schichtbereiche entfernt werden. Man erhält eine Druckform, die im Halbtonkeil 4 offene Keilstufen aufweist.

## Patentansprüche

**1.** Verbindungen der allgemeinen Formel I

$$R^2-CH_2-\overset{\overset{D}{|}}{N}-CH_2-R^1-OH \qquad (I)$$

worin

D einen 1,2-Naphthochinon-2-diazid-4-sulfonyl- oder -5-sulfonylrest,

$R^1$ eine Alkylengruppe mit 1 bis 3 Kohlenstoffatomen,

$R^2$ ein Wasserstoffatom, eine Alkylgruppe mit 1 bis 3 Kohlenstoffatomen oder eine Gruppe $R^1$-OH bedeutet,

oder der allgemeinen Formel II

$$\left[-O-R^1-CH_2-\overset{D}{\overset{|}{N}}-CH_2-R^1-O-\right]_n \left[-CO-X-CO-\right]_{n+m-1} \left[-O-R^3-O-\right]_m \quad (II)$$

worin

X eine Alkylengruppe mit 2 bis 12 Kohlenstoffatomen, eine Arylengruppe mit 6 bis 10 Kohlenstoffatomen oder eine Gruppe der Formel

NH-Y-NH,

worin

Y eine Alkylengruppe mit 2 bis 12 Kohlenstoffatomen oder eine Arylengruppe mit 6 bis 14 Kohlenstoffatomen ist,

$R^3$ eine Alkylengruppe mit 2 bis 12 Kohlenstoffatomen, die ggf. durch Ethersauerstoffatome unterbrochen ist,

n eine Zahl von 1 bis 40 und

m eine Zahl von 0 bis 50 bedeutet,

wobei das Verhältnis m:(m+n) 0:100 bis 95:100 beträgt und $R^1$ und D die oben angegebene Bedeutung haben.

2. Verbindungen nach Anspruch 1, dadurch gekennzeichnet, daß Y eine Alkylengruppe mit 6 bis 10 Kohlenstoffatomen ist.

3. Verbindungen nach Anspruch 1, dadurch gekennzeichnet, daß $R^3$ eine Alkylengruppe mit 4 bis 8 Kohlenstoffatomen ist, die ggf. durch Ethersauerstoffatome unterbrochen ist.

4. Verbindungen nach Anspruch 1, dadurch gekennzeichnet, daß das Verhältnis m:(m+n) 10:100 bis 95:100 beträgt.

5. Lichtempfindliches Gemisch, das als wesentliche Bestandteile ein polymeres, wasserunlösliches, in organischen Lösemitteln und in wäßrig-alkalischen Lösungen lösliches Bindemittel und ein 1,2-Naphthochinon-2-diazid-sulfonsäureamid enthält, dadurch gekennzeichnet, daß das 1,2-Naphthochinon-2-diazid-sulfonsäureamid eine Verbindung gemäß einem der Ansprüche 1 bis 4 ist.

6. Lichtempfindliches Gemisch nach Anspruch 5, dadurch gekennzeichnet, daß es 5 bis 100 Gew.-%, bezogen auf seine nichtflüchtigen Bestandteile, an Verbindungen gemäß Anspruch 1 enthält.

7. Lichtempfindliches Aufzeichnungsmaterial mit einem Schichtträger und einer lichtempfindlichen Schicht, dadurch gekennzeichnet, daß die lichtempfindliche Schicht eine Verbindung gemäß einem der Ansprüche 1 bis 4 enthält.

## Claims

1. Compounds of the general formula I

$$R^2-CH_2-\overset{D}{\overset{|}{N}}-CH_2-R^1-OH \quad (I)$$

in which

D denotes a 1,2-naphthoquinone-2-diazide-4-sulfonyl or -5-sulfonyl radical,

$R^1$ denotes an alkylene group with 1 to 3 carbon atoms and

$R^2$ denotes a hydrogen atom, an alkyl group with 1 to 3 carbon atoms or a group $R^1$-OH,

or of the general formula II

$$\left[-O-R^1-CH_2-\overset{D}{\underset{|}{N}}-CH_2-R^1-O-\right]_n \left[-CO-X-CO-\right]_{n+m-1} \left[-O-R^3-O-\right]_m \quad (II)$$

in which

X denotes an alkylene group with 2 to 12 carbon atoms, an arylene group with 6 to 10 carbon atoms or a group of the formula

NH-Y-NH,

wherein

Y is an alkylene group with 2 to 12 carbon atoms or an arylene group with 6 to 14 carbon atoms,

$R^3$ denotes an alkylene group with 2 to 12 carbon atoms, which may be interrupted by ether oxygen atoms,

n is a number from 1 to 40 and

m is a number from 0 to 50,

the ratio m:(m+n) being from 0:100 to 95:100 and $R^1$ and D having the above-indicated significations.

2. The compounds as claimed in claim 1, wherein Y is an alkylene group having 6 to 10 carbon atoms.

3. The compounds as claimed in claim 1, wherein $R^3$ is an alkylene group having 4 to 8 carbon atoms, which may be interrupted by ether oxygen atoms.

4. The compounds as claimed in claim 1, wherein the ratio m:(m+n) is from 10:100 to 95:100.

5. A photosensitive composition which comprises, as the essential components, a polymeric binder which is insoluble in water and soluble in organic solvents and in aqueous-alkaline solutions and a 1,2-naphthoquinone-2-diazide sulfonic acid amide, wherein the 1,2-naphthoquinone-2-diazide sulfonic acid amide is a compound as claimed in any of claims 1 to 4.

6. A photosensitive composition as claimed in claim 5, which contains 5 to 100 % by weight, based on its non-volatile components, of compounds as claimed in claim 1.

7. A photosensitive recording material which comprises a support and a photosensitive layer, wherein the photosensitive layer contains a compound as claimed in any of claims 1 to 4.

## Revendications

1. Composés de formule générale I

$$R^2-CH_2-\overset{D}{\underset{|}{N}}-CH_2-R^1-OH \quad (I)$$

dans laquelle

D représente un radical 1,2-naphtoquinone-2-diazido-4-sulfonyle ou -5-sulfonyle,

$R^1$ représente un groupe alkylène ayant de 1 à 3 atomes de carbone,

$R^2$ représente un atome d'hydrogène, un groupe alkyle ayant de 1 à 3 atomes de carbone, ou un groupe $R^1$-OH,

ou de formule générale II

$$\left[-O-R^1-CH_2-\overset{\displaystyle D}{\overset{|}{N}}-CH_2-R^1-O-\right]_n \left[-CO-X-CO-\right]_{n+m-1} \left[-O-R^3-O-\right]_m \quad (II)$$

dans laquelle

X représente un groupe alkylène ayant de 2 à 12 atomes de carbone, un groupe arylène ayant de 6 à 10 atomes de carbone ou un groupe de formule

NH-Y-NH,

Y représentant un groupe alkylène ayant de 2 à 12 atomes de carbone ou un groupe arylène ayant de 6 à 14 atomes de carbone,

$R^3$ représente un groupe alkylène ayant de 2 à 12 atomes de carbone, qui est éventuellement interrompu par des atomes d'oxygène en fonction éther,

n est un nombre entier allant de 1 à 40, et

m est un nombre entier allant de 0 à 50,

le rapport m:(m+n) allant de 0:100 à 95:100 et $R^1$ et D ayant les significations données plus haut.

**2.** Composés selon la revendication 1, caractérisés en ce que Y est un groupe alkylène ayant de 6 à 10 atomes de carbone.

**3.** Composés selon la revendication 1, caractérisés en ce que $R^3$ est un groupe alkylène ayant de 4 à 8 atomes de carbone, qui est éventuellement interrompu par des atomes d'oxygène en fonction éther.

**4.** Composés selon la revendication 1, caractérisés en ce que le rapport m:(m+n) va de 10:100 à 95:100.

**5.** Composition photosensible qui contient, en tant que composants essentiels, un liant polymère insoluble dans l'eau, soluble dans des solvants organiques et dans des solutions aqueuses-alcalines, et un 1,2-naphtoquinone-2-diazido-sulfonamide, caractérisée en ce que le 1,2-naphtoquinone-2-diazido-sulfonamide est un composé selon l'une des revendications 1 à 4.

**6.** Composition photosensible selon la revendication 5, caractérisée en ce qu'elle contient de 5 à 100 % en poids, par rapport à ses composants non volatils, de composés selon la revendication 1.

**7.** Matériau de reprographie photosensible, comportant un support de couche et une couche photosensible, caractérisé en ce que la couche photosensible contient un composé selon l'une des revendications 1 à 4.